# EUROPEAN PATENT SPECIFICATION

(11) **EP 3 645 087 B1**
(45) Date of publication and mention of the grant of the patent: **26.05.2021**
(21) Application number: 18733835.5
(22) Date of filing: 20.06.2018
(51) Int. Cl.: A61M 11/00, A61M 15/00, A61M 16/16, A61M 16/08

(54) **INSERT WITH AEROSOL GENERATOR FOR A SYSTEM FOR HUMIDIFICATION OF A PRESSURIZED FLOW OF BREATHABLE GAS DELIVERED TO A PATIENT**
EINSATZ MIT AEROSOLERZEUGER FÜR EIN SYSTEM ZUR BEFEUCHTUNG EINES UNTER DRUCK STEHENDEN DURCHFLUSSES VON ATEMGAS, DAS EINEM PATIENTEN BEREITGESTELLT WIRD
INSERT AVEC GÉNÉRATEUR D'AÉROSOL POUR UN SYSTÈME D'HUMIDIFICATION D'UN FLUX PRESSURISÉ DE GAZ RESPIRABLE DÉLIVRÉ À UN PATIENT

(30) Priority: 28.06.2017 EP 17178370
(43) Date of publication of application: 06.05.2020
(73) Proprietor: Koninklijke Philips N.V., 5656 AG Eindhoven (NL)
(72) Inventor: HILBIG, Rainer, 5656 AE Eindhoven (NL); DE GRAAF, Pascal, 5656 AE Eindhoven (NL); MULDER, Marcel, 5656 AE Eindhoven (NL); KOERBER, Achim, Gerhard, Rolf, 5656 AE Eindhoven (NL)
(74) Representative: Philips Intellectual Property & Standards
(86) International application number: PCT/EP2018/066332
(87) International publication number: WO 2019/002033

(56) References cited:
- EP-A1- 1 066 850
- EP-A1- 3 037 120
- EP-A2- 1 818 070
- WO-A1-01/19437
- WO-A1-2015/196379
- WO-A1-2018/109095
- DE-A1-102008 022 987
- JP-A- 2005 058 709
- US-A1- 2016 279 351

## Description

### FIELD OF THE INVENTION

The present invention relates to a system for humidification of a pressurized flow of breathable gas delivered to a patient. The invention further relates to an insert for use in such a system.

### BACKGROUND OF THE INVENTION

Patients with invasive ventilation delivered for example through a patient's trachea experience the situation that if dry ventilated air is delivered to them via a ventilator, the airways are drying out. Drying out of the upper airways due to inhalation of dry air over some period, is uncomfortable for the patient and can have a severe health impact after longer exposure. Inhalation of dry air over a longer period results in a drying of mucus in the upper airways, causing it to become very viscous, impeding mucus removal from the patient's airway. Excess mucus in the upper airways negatively effects the breathing of the patients and thus their quality of life, and if not removed poses a risk of inflammations.

To support patients with non-invasive ventilation, e.g. severe chronic obstructive pulmonary disease (COPD) patients or patients with neuromuscular disorders, next to the ventilator a humidifier is supplied that humidifies the ventilated air.

US20090235925 discloses a system for humidification of a pressurized flow of breathable gas delivered to a patient having a ventilator, a patient circuit and an aerosol generator that is connected to the patient circuit by means of a connector. Such systems for humidification of a pressurized flow of breathable gas delivered to a patient of this general type may, however, be further improved.

JP2005058709 discloses a humidifier for an artificial respirator comprising: a water tank attached to a main body of the humidifier arranged at a patient's side; a water supplying member supplying water into the humidifier main body; an ultrasonic vibration plate having multiple pores and touching the water supplying member; an ultrasonic transducer connected to the ultrasonic vibration plate; a control instrument connected to the ultrasonic transducer; detection means detecting pressure and temperature in the humidifier main body; and a heating means located near the ultrasonic transducer.

EP1066850A1 discloses a medical nebulizer comprises a reservoir for medical liquid; a capillary nozzle having a proximal end for receiving liquid from the reservoir and a distal end connectable to an inspiration gas flow path to deliver liquid droplets into an inspiration gas flow. The nebulizer further comprises pump means for supplying liquid from the reservoir through the capillary nozzle, and means for regulating the delivery of the liquid to a predetermined amount.

EP3037120A1 discloses an aerosol delivery device comprising an aerosol generator for generating an aerosol in the aerosol delivery device, a sensor configured to detect a use of the aerosol delivery device for aerosol treatment, and a controller configured to deactivate the aerosol generator if no use of the aerosol delivery device for aerosol treatment is detected by the sensor.

US2016279351A1 discloses a fluid reservoir which is attachable to an aerosol generation device for guiding a fluid from a fluid container to the aerosol generation device. The fluid reservoir has an interface portion arranged at the fluid reservoir for attaching the fluid reservoir to the aerosol generation device. The interface portion has a locking element configured to non-detachably lock the fluid reservoir to the aerosol generation device after attachment of the fluid reservoir to the aerosol generation device. The locking element is breakable to enable detachment of the fluid reservoir from the aerosol generation device.

WO2015196379A1 discloses a micro-humidifier for operably-connecting to a breathing circuit. It contains a liquid feed, a liquid chamber operably-connected to the liquid feed, and a vapour generator operably-connected to the liquid chamber. The micro-humidifier is operably-connected to and/or is designed to be operably-connected to a breathing circuit upstream of the patient.

WO0119437A1 discloses a nebulizer apparatus to atomize liquid solutions or suspensions. The nebulizer is typically used in conjunction with a breathing circuit to deliver atomized medicine to a patient. A housing with an opening covered by a thin mesh plate is supplied with the liquid to be nebulized. The mesh plate or liquid is vibrated at ultrasonic frequencies to atomize the liquid as it passes through the plate into breathing gases flowing through the breathing tube.

DE102008022987A1 discloses a nebulizer for a ventilator. The ventilator comprises a body with a first connector for connecting the nebulizer to a ventilator and a second connector for connecting the nebulizer to a patient-carrying line. The body forms a flow channel from the first connection to the second connection.

EP1818070A2 discloses an inhalation therapy device which includes an aerosol creating device, a breathing air flow creating device and an atomizing chamber. The intubation device is so arranged that the intubation end can be positioned to ensure that the droplet-mist and breathing air mixture only goes to regions of the breathing passage from which they will be filtered out.

### SUMMARY OF THE INVENTION

The invention is defined by the claims.

According to examples in accordance with an aspect of the invention, there is provided a system for humidification of a pressurized flow of breathable gas delivered to a patient, in particular a system which can be used without the risk of liquid water getting into the patient's airways (rainout).

Towards this end, a first aspect of the invention provides a system for humidification of a pressurized flow of breathable gas delivered to a subject, the system comprising:
a ventilator for generating a pressurized flow of breathable gas;
a patient circuit in fluid communication with the ventilator and connectable to the respiratory system of a patient, the patient circuit defining an internal space for transportation of the breathable gas;
an aerosol generator for providing an aerosol of liquid, the aerosol generator having an outflow opening.

The system further comprises an insert that is connectable to the patient circuit and that accommodates the aerosol generator. This enables an easy and straightforward way of mounting the aerosol generator. It even allows to easily change between a regular ventilating system and a humidification system. More in particular, the insert in the direction of the flow of breathable gas first expands and then contracts to enclose the aerosol generator. This provides an insert with of relatively limited size. To accommodate the aerosol generator and in particular its connections the expanding part comprises an open space.

The outflow opening of the aerosol generator debouches in an internal space of the insert for entrainment of the aerosol of liquid.

The internal space of the patient circuit is used to transport the breathable gas from the ventilator to the connection with the respiratory system of a patient. Providing the outflow opening of the aerosol generator within the internal space of the insert enables a direct entrainment of the aerosol in the flow of breathable gas and with that prevents rain out, or at least reduces the chance to rainout. The aerosol generator can be positioned closer to the patient which further reduces the chance for condensation and thus rain out to occur. The system allows for an optimal mixing of the aerosolized liquid with the breathable gas.

In an arrangement, the aerosol generator is a nebulizer, more in particular a vibrating mesh nebulizer. Nebulizers are known and widely used to provide aerosols or aerosolized drugs. These are relatively silent, portable and small. Mesh nebulizers are particularly advantageous to nebulize or aerosolize aqueous solutions.

In another arrangement, the system further comprises a control unit to control at least the ventilator and the aerosol generator, wherein in the control unit is configured to control the amount and humidity of the breathable gas based on a patient's needs. This enhances the flexibility and allows a better patient comfort.

### BRIEF DESCRIPTION OF THE DRAWINGS

Examples of the invention will now be described in detail with reference to the accompanying drawings, in which:
Fig. 1 shows a schematic view of a known system for humidification of a pressurized flow of breathable gas delivered to a patient.
Fig. 2 shows a schematic view of a system for humidification of a pressurized flow of breathable gas delivered to a patient according to an example arrangement.
Fig. 3 shows a schematic view of an insert according to an embodiment of the invention that is used in a system for humidification of a pressurized flow of breathable gas delivered to a patient.

### DETAILED DESCRIPTION OF EMBODIMENTS

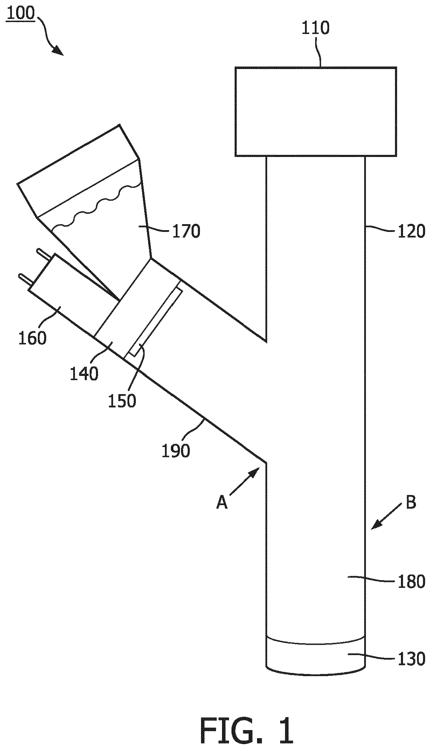

As noted above, Fig. 1 shows a schematic view of a known system 100 for humidification of a pressurized flow of breathable gas delivered to a patient. The system 100 comprises a ventilator 110, a patient circuit 120 in fluid communication with the ventilator. The patient circuit has an internal space 180 for transportation of the breathable gas. At an end of the patient circuit a connector 130 is provided that is used to connect the patient circuit with the respiratory system of a patient. The system 100 further comprises an aerosol generator 140 having an outflow opening 150. The aerosol generator is connected to a liquid reservoir 170. A connection unit 160 is provided for the (electrical) connections of the aerosol generator. A connector 190 is provided to connect the outflow opening 150 of the aerosol generator with the patient circuit 120. At the locations indicated with arrows A and B there is a high risk for condensation to occur due to the collision of the small water droplets. Further on, the water droplets are created in a kind of dead volume that is not effectively flushed by the air delivered by the ventilator 110 towards the patient circuit connector 130. Therefore, the amount of droplets delivered to the patient per time unit is not well defined.

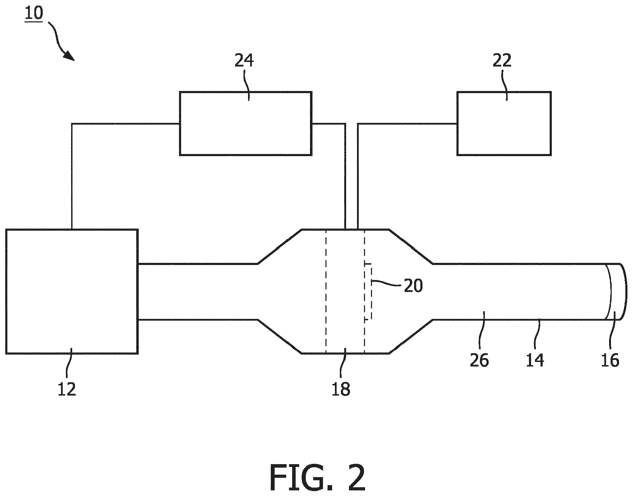

For purposes of better understanding embodiments of the invention, Fig. 2 shows a schematic view of an example system 10 for humidification of a pressurized flow of breathable gas delivered to a patient. The system 10 comprises a ventilator 12, a patient circuit 14 in fluid communication with the ventilator. At and end of the patient circuit a connector 16 is provided that is used to connect the patient circuit with the respiratory system of a patient. The patient circuit has an internal space 26 for transportation of the breathable gas. The system, as illustrated in Fig. 2, further comprises an aerosol generator 18 having an outflow opening 20, both indicated with dotted lines. The outflow opening 20 is provided within the internal space 26 allowing a direct entrainment of the aerosol of liquid with the breathable gas.

Preferably the ventilator uses a turbine to generate the air flow towards the patient.

The system further comprises a control unit 24 that is connected to the ventilator 12 and the aerosol generator 18. The control unit preferably is configured to control the amount and humidity of the breathable gas based on a patient' needs. The control unit preferably arranges for the system to work only during (part of) the inhalation phase of the breathing cycle of a patient. Information on the breathing cycle to trigger on can be got, from the ventilator settings in case a ventilator that measures the breath rate is used.

The aerosol generator 18 is connected to a liquid reservoir 22.

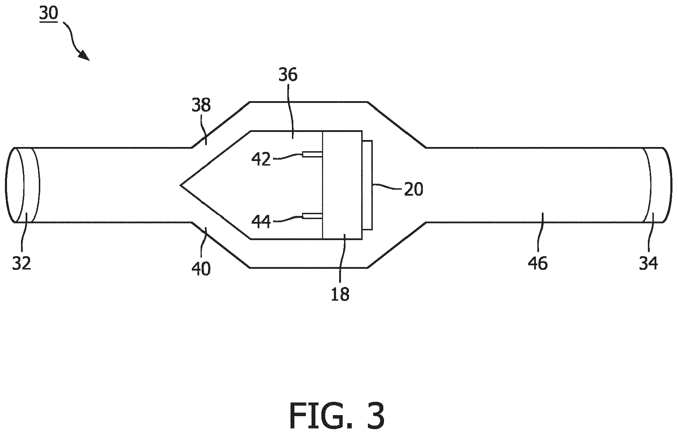

As noted above, Fig. 3 shows a shows a schematic view of an insert 30 in accordance with an embodiment of the invention that is used in a system for humidification of a pressurized flow of breathable gas delivered to a patient as shown in Fig. 2. The insert at both ends comprises connectors 32 and 34 for easy connecting with or mounting to a patient (air) circuit. The flow direction of breathable gas runs from connector 32 at the left side to the connector 34 at the right side of the drawing. In this direction the insert first expands and then contracts. In this way space is made available to accommodate the aerosol generator 18. More particular the air tube first bifurcates into 2 separate parts 38 and 40 and then rejoins into a single part. The outflow opening 20 of the aerosol generator 18 debouches in the internal space 46 of the insert. The insert further comprises an open space 36 provided in the expanding part this allows the space for mounting of the aerosol generator 18. The aerosol generator 18 is mounted in the open space, 36, wherein the outflow opening 20 coincides with an opening in the outer wall of the insert 30. It also provides space for an electrical connection 42 and a connection to the liquid water reservoir 44 of the aerosol generator.

The aerosol generator ideally is placed as close to the patient as possible to avoid water losses in the circuit. The present invention enables such placement of due to the advantageous connection between the patient circuit and the aerosol generator.

Sterile water or sterile normal saline preferably is used as humidifying agent. Preferably the system is sealed from the atmosphere to reduce contamination risks. The breathable gas preferably is regular air.

Connection to the respiratory system of a patient can be obtained in a number of ways, for example one can connect the insert to an endotracheal tube for intubated patients. Other possibilities are connection via a nasal of facial mask. It is also possible to use the system in combination with a device delivering a positive pressure (CPAP-) for treating sleep apnea.

The system can be advantageoulsy used for a whole range of patients that have breathing difficulties with their respiratory system, such as chronic obstructive pulmonary disease (COPD) patients, asthma patients or patients with neuromuscular disorders.

Experiments have been performed to demonstrate the improvement of the design as shown in Figs. 2 and 3 in comparison with the design shown in Fig. 1. The patient respiratory system was replaced by an artificial lung with a volume of 0.6 litre. The peak flow of the ventilator (during inhalation phase) was set at 70 L/min and a breathing cycle with 1 s for inhalation and with 2 s for exhalation was used. Further an amount of up to 33 mg of water per liter air generated by the nebulizer was used. It was found that the system according to the invention shows proper mixing of air with the water droplets and no rainout. Running the known system under same conditions resulted in worse mixing of air and water and rainout that is clearly visible in the regions indicated with arrows A and B of the known system as shown in Fig. 1.

During operation of the system a power of <∼ 2 W is consumed. The existing active humidification devices wherein humidifaction is based on heating of water need more than an order of magnitude higher power. The relative low power comsumption and the efficient design -for example in terms of relative size- allows for a system that is battery operated and portable. Portable systems of course greatly enhance the comfort and flexibility of a patient.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. An insert (30) for use in a system (10) for humidification of a pressurized flow of breathable gas delivered to a patient,
said insert being connectable to a patient circuit (14) and accommodating an aerosol generator (18) for providing an aerosol of liquid, the aerosol generator having an outflow opening;
said insert defining an internal space (46), and the outflow opening being arranged to debouch into the internal space (46) of the insert for entrainment of the aerosol of liquid;
said insert (30) first expanding in the direction of the flow of breathable gas, thus defining an expanding part, and then contracting to enclose the aerosol generator (18), **characterized in that** said insert comprises an air tube that in the direction of the flow of breathable gas first bifurcates into 2 separate parts (38, 40) and then rejoins into a single part, wherein the expanding part comprises an open space (36) for accommodating the aerosol generator (18).

2. A system (10) for humidification of a pressurized flow of breathable gas delivered to a patient, the system comprising:
a ventilator (12) for generating a pressurized flow of breathable gas;
a patient circuit (14) in fluid communication with the ventilator and connectable to the respiratory system of a patient, the patient circuit defining an internal space (26) for transportation of the breathable gas; and
an insert (30) in accordance with claim 1, the insert being connectable to the patient circuit (14).

3. The system (10) according to claim 2, wherein the aerosol generator (18) is connected to a liquid reservoir (22).

4. The system (10) according to claim 2 or 3, wherein the aerosol generator (18) is a nebulizer, preferably a vibrating mesh nebulizer.

5. The system (10) according to any of claims 2-4, wherein the system further comprises a control unit (24) to control at least the ventilator (12) and the aerosol generator (18), wherein in the control unit is configured to control the amount and humidity of the breathable gas based on a patient' needs.

6. The system (10) according to any of claims 2-5, wherein the system is a battery operated portable system.

## Patentansprüche

1. Ein Einsatz (30) für ein System (10) zum Befeuchten eines mit Druck beaufschlagten Atemluftstroms, der einem Patienten zugeführt wird,
wobei der Einsatz an einen Patientenkreislauf (14) angeschlossen werden und einen Aerosolgenerator (18) zum Bereitstellen eines Flüssigkeitsaerosols aufnehmen kann,
wobei der Aerosolgenerator eine Ausflussöffnung umfasst; wobei der Einsatz einen Innenraum (46) definiert, und wobei die Ausflussöffnung so angeordnet ist, dass sie in den Innenraum (46) des Einsatzes mündet, um das Flüssigkeitsaerosol mit sich zu führen; wobei sich der Einsatz (30) zunächst in Richtung des Atemluftstroms ausdehnt, um einen sich ausdehnenden Abschnitt zu definieren, während er sich anschließend zusammenzieht, um den Aerosolgenerator (18) zu umschließen, der sich dadurch auszeichnet, dass der Einsatz eine Luftröhre umfasst, die sich in Richtung des Atemluftstroms zunächst in 2 getrennte Teile (38, 40) gabelt um sich anschließend wieder zu einem einzigen Teil zusammenzufügen, wobei der sich ausdehnende Teil einen offenen Raum (36) zur Aufnahme des Aerosolgenerators (18) aufweist.

2. Ein System (10) zum Befeuchten eines mit Druck beaufschlagten Atemluftstroms, der einem Patienten zugeführt wird, wobei das System Folgendes umfasst:
ein Beatmungsgerät (12) zum Erzeugen eines mit Druck beaufschlagten Atemluftstroms;
einen Patientenkreislauf (14), der über eine Fluidverbindung zum Beatmungsgerät verfügt und an das Atmungssystem eines Patienten angeschlossen werden kann, wobei der Patientenkreislauf einen Innenraum (26) zum Befördern der Atemluft definiert; und
einen Einsatz (30) gemäß Anspruch 1, wobei der Einsatz mit dem Patientenkreislauf (14) verbunden werden kann.

3. Das System (10) gemäß Anspruch 2, wobei der Aerosolgenerator (18) mit einem Flüssigkeitsreservoir (22) verbunden ist.

4. Das System (10) gemäß Anspruch 2 oder 3, wobei es sich beim Aerosolgenerator (18) um einen Zerstäuber und vorzugsweise um einen vibrierenden Mesh-Zerstäuber handelt.

5. Das System (10) gemäß einem der Ansprüche 2 bis 4, wobei das System zudem eine Steuerungseinheit (24) umfasst, um mindestens das Beatmungsgerät (12) und den Aerosolgenerator (18) zu steuern, wobei die Steuerungseinheit die Menge und die Feuchtigkeit der Atemluft beruhend auf den Bedürfnissen des Patienten steuert.

6. Das System (10) gemäß einem der Ansprüche 2 bis 5, wobei es sich beim System um ein batteriebetriebenes tragbares System handelt.

## Revendications

1. Insert (30) destiné à être utilisé dans un système (10) d'humidification d'un écoulement sous pression de gaz respirable distribué à un patient,
ledit insert pouvant être connecté à un circuit patient (14) et recevant un générateur d'aérosol (18) pour fournir un aérosol de liquide, le générateur d'aérosol comportant une ouverture de sortie ;
ledit insert définissant un espace interne (46), et l'ouverture de sortie étant conçue pour déboucher dans l'espace interne (46) de l'insert pour entraîner l'aérosol de liquide ;
ledit insert (30) se dilatant d'abord dans la direction due l'écoulement de gaz respirable, définissant ainsi une partie dilatante, puis se contractant pour enfermer le générateur d'aérosol (18), **caractérisé en ce que** ledit insert comprend un tube d'air, lequel, dans la direction de l'écoulement de gaz respirable, se bifurque d'abord en 2 parties (38, 40) séparées puis se rejoint en une seule partie, dans lequel la partie dilatante comprend un espace ouvert (36) destinée à la réception du générateur d'aérosol (18).

2. Système (10) d'humidification d'un écoulement sous pression de gaz respirable distribué à un patient, ledit système comprenant :
un ventilateur (12) destiné à la génération d'un écoulement sous pression de gaz respirable ;
un circuit patient (14) en communication fluidique avec le ventilateur et pouvant être relié au système respiratoire d'un patient, le circuit patient définissant un espace interne (26) destiné au transport du gaz respirable ; et
un insert (30) selon la revendication 1, l'insert pouvant être relié au circuit patient (14).

3. Système (10) selon la revendication 2, dans lequel le générateur d'aérosol (18) est relié à un réservoir de liquide (22).

4. Système (10) selon la revendication 2 ou 3, dans lequel le générateur d'aérosol (18) est un nébuliseur, de préférence un nébuliseur à tamis vibrant.

5. Système (10) selon l'une quelconque des revendications 2 à 4, dans lequel ledit système comprend en outre une unité de commande (24) destinée à la commande d'au moins le ventilateur (12) et le générateur d'aérosol (18), dans lequel l'unité de commande est conçue pour contrôler la quantité et l'humidité du gaz respirable en fonction des besoins d'un patient.

6. Système (10) selon l'une quelconque des revendications 2 à 5, dans lequel le système est un système portable fonctionnant sur batterie.
